# EUROPEAN PATENT SPECIFICATION

(11) **EP 2 993 168 B1**
(45) Date of publication and mention of the grant of the patent: **17.05.2017**
(21) Application number: 15181106.4
(22) Date of filing: 24.10.2005
(51) Int. Cl.: C07D 211/44, C07D 211/94, C07D 251/44, C07D 251/50, C07D 251/54, C08K 5/3435, C08K 5/3492, C09K 15/20

(54) **PROCESS FOR THE SYNTHESIS OF N-ALKOXYAMINES**
VERFAHREN ZUR SYNTHESE VON N-ALKOXYAMINEN
PROCÉDÉ POUR LA SYNTHÈSE DE N-ALKOXYAMINES

(30) Priority: 02.11.2004 EP 04105456
(43) Date of publication of application: 09.03.2016
(62) Divisional of application: 05801650.2
(73) Proprietor: BASF SE, 67056 Ludwigshafen am Rhein (DE)
(72) Inventor: Frey, Markus, 4310 Rheinfelden (CH); Broennimann, Valérie, 4058 Basel (CH); Martinez, Francisco, 43100 Parma (IT); Alvisi, Davide, 44012 Bondeno (FE) (IT)
(74) Representative: BASF IP Association

(56) References cited:
- WO-A-03/045919
- US-A- 5 204 473
- US-B1- 6 265 473

## Description

The present invention relates to novel processes for the preparation of a sterically hindered amine ether by the transformation of a corresponding oxo-piperidin to a hydroxy or animo substituted sterically hindered amine ether and the preparation of a N-propoxy or N-propenoxy substituted sterically hindered amine and some novel compounds obtainable by these processes. The compounds made by these processes are particularly effective in the stabilization of polymer compositions against harmful effects of light, oxyen and/or heat and as flame-retardants for polymers.

WO 01/92228 describes a process for the preparation of amine ethers, e.g. N-hydrocarbyloxy substituted hindered amine compounds, by the reaction of the corresponding N-oxyl intermediate with a hydrocarbon in the presence of an organic hydroperoxide and a copper catalyst.

WO 03/045919 describes a process for the preparation of amine ethers, e.g. N-hydrocarbyloxy substituted hindered amine compounds, by the reaction of the corresponding N-oxyl intermediate with a hydrocarbon in the presence of an organic hydroperoxide and an iodide catalyst.

DE19907945A describes the formation of 1-allyloxy substituted sterically hindered amines from 1-allyl substituted sterically hindered amines by oxidation.

WO 98/54174 and US 5,844,026 describe the reductive amination of a N-cyclohexyloxy-2,2,6,6-tetramethyl-4-oxo-piperidine to the corresponding amine.

A problem of the state of the art processes is that undesirable side products are obtained that are hard to remove from the desired products as amine oxides do not react selectively with saturated hydrocarbons. The processes of the present invention avoid this problem as hydrocarbons with unsaturated carbon-carbon bonds react more selectively than saturated hydrocarbons, i.e. compounds prepared according to the instant processes may be purer. The transformation product of the process of the present invention may easily be purified by standard methods such as distillation. The hydrogenation of the unsaturated carbon-carbon bond and the reduction or reductive amination of the carbonyl group in one reation step may save one reaction step and may need less solvents and reagents than the state of the art, i.e. this reaction preformed in two separate reaction steps.

This invention relates to a process for the preparation of a compound of formula (300) 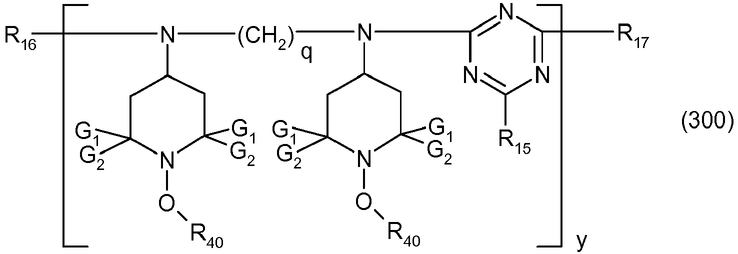 wherein
G₁ and G₂ are independently C₁-C₄alkyl;
R₄₀ is propyl or 2-propenyl;
y is 2 to 20;
q is 2 to 8;
R₁₅ is morpholino, piperidino, 1-piperizinyl, alkylamino of 1 to 8 carbon atoms, -N(C₁-C₈alkyl)T₁₀, or -N(alkyl)₂ of 2 to 16 carbon atoms, 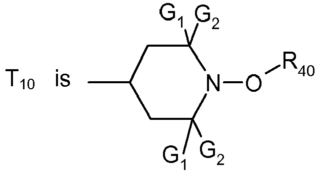
R₁₆ is hydrogen, C₂-C₄acyl, carbamoyl substituted by C₁-C₄alkyl, s-triazinyl substituted once by chlorine and once by R₁₅, or s-triazinyl substituted twice by R₁₅ with the condition that the two R₁₅ substituents may be different;
R₁₇ is chlorine, amino substituted by C₁-C₈alkyl or by T₁₀, -N(C₁-C₈alkyl)T₁₀, -N(alkyl)₂ of 2 to 16 carbon atoms, or the group T₁₃ 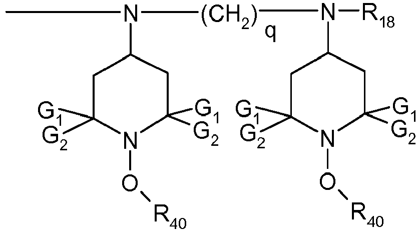 R₁₈ is hydrogen, C₂-C₄acyl, carbamoyl substituted by C₁-C₄alkyl, s-triazinyl substituted twice by -N(alkyl)₂ of 2 to 16 carbon atoms or s-triazinyl substituted twice by -N(C₁-C₈alkyl)T₁₀;
which comprises oxidizing a compound of formula (300) wherein >N-O-R₄₀ is >N-H to a compound of formula (300) wherein -O-R₄₀ is -O•, which is subsequently reacted with propene;
and hydrogenating this compound for obtaining a compound of formula (300) with R₄₀ = propyl.

Of technical interest are compounds of formula (300) wherein R₁₅ is -N(C₁-C₈alkyl)T₁₀, R₁₆ is s-triazinyl substituted twice by R₁₅ = -N(alkyl)₂ of 2 to 16 carbon atoms, R₁₇ is T₁₃, R₁₈ is s-triazinyl substituted twice by -N(alkyl)₂ of 2 to 16 carbon atoms.

Starting compounds of formula (300) wherein >N-O-R₄₀ is >N-H are known in the art, are partly commercially available or can be synthesised according to procedures known in the art as for example described in DE19959619 or CA2191832.

Advantageously, hydrogenation of compound of formula (300) with R₄₀ = propenyl is carried out in the presence of a hydrogenation catalyst.

The hydrogenation catalyst is preferably selected from the group consisting of platinum, palladium, ruthenium, rhodium, Lindlar catalyst, platinum compounds, palladium compounds, ruthenium compounds, rhodium compounds, iridium compounds, nickel compounds, zinc compounds and cobalt compounds.

The hydrogenation catalyst can be bound to an organic or inorganic polymer backbone, rendering a homogenous or heterogeneous catalytic system. Hydrogenation can also be carried out as transfer hydrogenation such as described in S. Murashi et al., Chem. Rev. (1998), 98, 2599-2660 or with further hydrogenation methods such as described in Larock, comprehensive organic transformations.

More preferably, the hydrogenation catalyst is selected from the group consisting of platinum, palladium, ruthenium, platinum compounds, palladium compounds and ruthenium compounds.

Most preferably, the hydrogenation catalyst is selected from the group consisting of platinum, palladium and ruthenium; platinum, palladium and ruthenium immobilized on carbon; PtO₂, Pd-CaCO₃-PbO, RuClH[PPh₃]₃, RhCl[PPh₃]₃ and RuH₂[P(Ph)₃]₄.

The preferred amount of hydrogenation catalyst is 0.0001-0.2 mol per mol of unsaturated amine ether moiety. The hydrogenation reaction is preferably run at 0° to 80°C; especially in the range 20-60°C. The hydrogen pressure is preferably 1-20 atm, for example 1-5 atm.

In the above-mentioned processes, G₁ and G₂ are for example methyl.

Some of the compounds available by the instant processes are of formula (403) and (404) 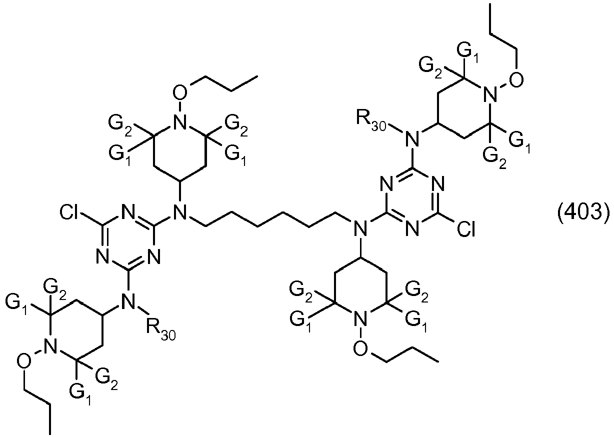 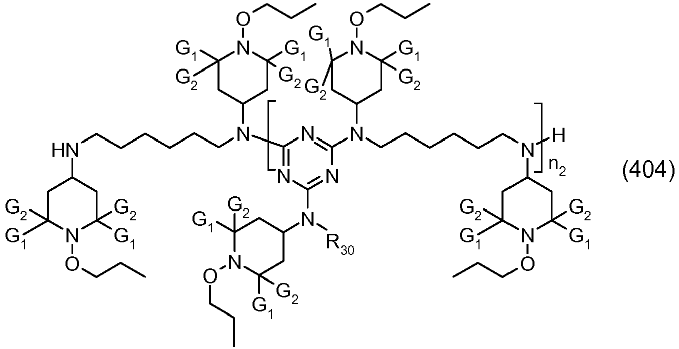
wherein G₁ and G₂ are independently C₁-C₄alkyl;
R₃₀ is C₁-C₈alkyl and
n₂ is 2 to 20.

The instant compounds may be prepared according to the process of this invention.

In the definitions the term alkene comprises, for example propene, and the branched and unbranched isomers of butene, pentene, hexene, heptene, octene, nonene, decene, undecene and dodecene. The term alkene also comprises residues with more than one double bond that may be conjugated or non-conjugated, for example may comprise one double bond.

Some examples of cycloalkene are cyclopentene, cyclohexene, methylcyclopentene, dimethylcyclopentene and methylcyclohexene. Cycloalkene may comprise more than one double bond that may be conjugated or non-conjugated, for example may comprise one double bond.

In the definitions the term alkyl comprises within the given limits of carbon atoms, for example methyl, ethyl, propyl, isopropyl, n-butyl, sec-butyl, isobutyl, tert-butyl, 2-ethylbutyl, n-pentyl, isopentyl, 1-methylpentyl, 1,3-dimethylbutyl, n-hexyl, 1-methylhexyl, n-heptyl, 2-methylheptyl, 1,1,3,3-tetramethylbutyl, 1-methylheptyl, 3-methylheptyl, n-octyl, 2-ethylhexyl, 1,1,3-trimethylhexyl, 1,1,3,3-tetramethylpentyl, nonyl, decyl, undecyl, 1-methylundecyl or dodecyl.

Examples of alkenyl are within the given limits of carbon atoms vinyl, allyl, and the branched and unbranched isomers of butenyl, pentenyl, hexenyl, heptenyl, octenyl, nonenyl, decenyl, undecenyl and dodecenyl. The term alkenyl also comprises residues with more than one double bond that may be conjugated or non-conjugated, for example may comprise one double bond.

Examples of alkylene are within the given limits of carbon atoms branched and unbranched isomers of vinylene, allylene, butylene, pentylene, hexylene, heptylene, octylene, nonylene, decylene, undecylene and dodecylene.

Some examples of cycloalkyl are cyclopentyl, cyclohexyl, methylcyclopentyl, dimethylcyclopentyl and methylcyclohexyl.

Some examples of cycloalkenyl are cyclopentenyl, cyclohexenyl, methylcyclopentenyl, dimethylcyclopentenyl and methylcyclohexenyl. Cycloalkenyl may comprise more than one double bond that may be conjugated or non-conjugated, for example may comprise one double bond.

Aryl is for example phenyl or naphthyl.

The term alkoxy may comprise within the limits of the given number of carbon atoms, for example methoxy and ethoxy and the branched and unbranched isomers of propoxy, butoxy, pentyloxy, hexyloxy, heptyloxy, octyloxy, nonyloxy, decyloxy, undecyloxy, dodecyloxy, tridecyloxy, tetradecyloxy, pentadecyloxy, hexadecyloxy, heptadecyloxy and octadecyloxy.

The term halogen may comprises chlorine, bromine and iodine; for example halogen is chlorine except in formula (203).

This invention also relates to the use of at least one compound or a mixture of compounds according to this invention as a stabilizer for an organic polymer against degradation by light, oxygen and/or heat or as flame retardant for an organic polymer.

For instance, this invention pertains to the use of at least one compound according to this invention as a stabilizer for an organic polymer against degradation by light, oxygen and/or heat or as flame retardant for an organic polymer.

For example, this invention pertains to the use of a mixture of compounds according to this invention as a stabilizer for an organic polymer against degradation by light, oxygen and/or heat or as flame retardant for an organic polymer.

This invention also relates to a process for flame retarding an organic polymer or stabilizing an organic polymer against degradation by light, oxygen and/or heat, which process comprises applying to or incorporating into said polymer at least one compound or a mixture of compounds according to this invention.

For instance, this invention pertains to a process for flame retarding an organic polymer or stabilizing an organic polymer against degradation by light, oxygen and/or heat, which process comprises applying to or incorporating into said polymer at least one compound according to this invention.

### Examples : the following example 13 only illustrates the present invention. The remaining examples are background examples helpful for the understanding.

### Abbreviations for NOR building blocks:

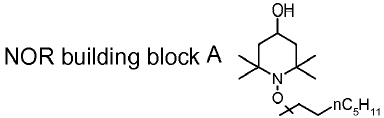

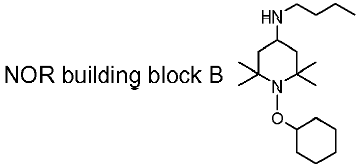

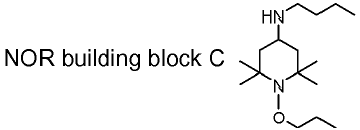

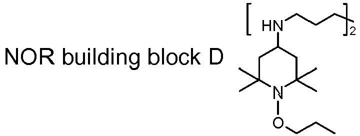

### Example 1: Preparation of NOR building block A in three steps from triacetonamine (TAA)

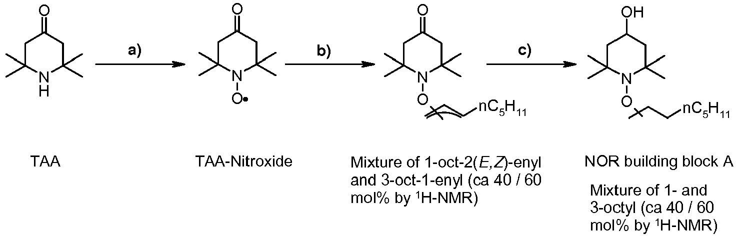

**a)** To a stirred mixture of 41.1 g (0.27 mol) triacetoneamine, 3.94 g (0.01 mol) sodium tungstate dihydrate and 250ml water are added at 5°C within 1 hour 64.9 g (0.57 mol) aqueous 30% hydrogenperoxide. The orange mixture is warmed to 25°C and stirring is continued for 21 hours. Potassium carbonate is then added until phase separation occurs and the triacetoneamine-N-oxide extracted three times with a total of 268 g (2.39 mol) 1-octene.
**b)** After addition of 0.64 g (4.8 mmol) cupric chloride the combined organic phases are brought to 60°C and 39.9 g (0.31 mol) t-butylhydroperoxide slowly dosed in. The mixture is held at 60°C for a total of 5.6 hours. The greenish suspension is then cooled to 25°C followed by the addition of 198 g aqueous 20% sodium sulfite. After stirring overnight the aqueous phase is split off and extracted with hexane. The combined organic phases are washed with water and concentrated on a rotary evaporator.
**c)** The residue is dissolved in 500 ml methanol, 9 g Ru on charcoal (5%) are added and the mixture hydrogenated at 50°C / 45 bar hydrogen during 17 hours. The mixture is filtered through hyflo and the filtrate concentrated on a rotary evaporator. Distillation of the residue yields 40.4 g (53.3%) of a slightly reddish oil (bp 123°C / 0.4 mbar) consisting of a mixture of 2,2,6,6-tetramethyl-1-octyloxy-piperidin-4-ol (ca 40 mol% by ¹H-NMR) and 1-(1-ethyl-hexyloxy)-2,2,6,6-tetramethyl-piperidin-4-ol (ca 60 mol% by ¹H-NMR).

Analysis required for C₁₇H₃₅NO₂ (285.47): C 71.53%, H 12.36%, N 4.91%; found: C 70.62%, H 12.69%, N 4.90%.
¹H-NMR (CDCl₃), δ (ppm, O-C(n)Hₓ only): 3.67 (p-like, O-C(3)H), 3.72 (t, J = ca 6.6 Hz, O-C(1)H₂), 3.95 (broad m, C(4)HOH from heterocycle).
¹³C(DEPT)-NMR (CDCl₃), δ (ppm, O-C(n)Hₓ only): 63.15 and 63.24 (C(4)HOH from heterocycle), 77.01 (O-C(1)H₂), 83.23 (O-C(3)H).

### Example 2: Synthesis of compound A' by transesterification of NOR building block A with sebacic acid dimethylester:

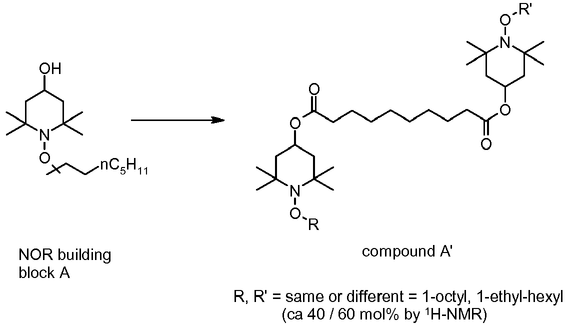

A mixture of 11.4 g (40 mmol) NOR building block A, 1.86 g (8 mmol) sebacic acid dimethylester and 0.135 g (1.6 mmol) LiOtBu are heated under vacuum (110°C, 200 mbar) during 22 hrs. The mixture is diluted with ethylacetate, washed pH neutral and the organic phase concentrated on a rotary evaporator. Flash chromatography (silica gel, hexane / ethylacetate 9 / 1) affords 4 g (68%) of the product as a slightly orange oil.

Analysis required for C₄₄H₈₄N₂O₆ (737.16): C 71.69%, H 11.49%, N 3.80%; found: C 71.47%, H 11.47%, N 3.69%.
¹H-NMR (CDCl₃), δ (ppm, O-C(n)Hₓ only): 3.67 (p-like, O-C(3)H), 3.72 (t, J = 6.8Hz, O-C(1)H₂).
¹³C(DEPT)-NMR (CDCl₃), δ (ppm, O-C(n)Hₓ only): 77.05 (O-C(1)H₂), 83.3 (O-C(3)H).

### Example 3: Preparation of NOR building block B from triacetonamine (TAA):

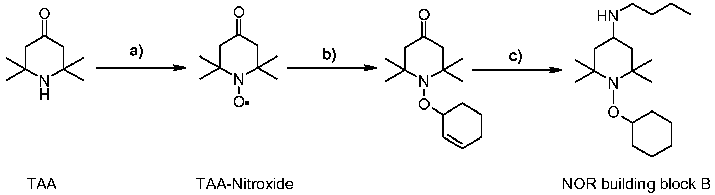

**a)** To a stirred mixture of 76.8g (0.49 mol) triacetoneamine, 7.1g (0.02 mol) sodium tungstate dihydrate and 445ml water are added at 5°C within 1 hour 122.5g (1.08 mol) aqueous 30% hydrogenperoxide. The orange mixture is warmed to 25°C and stirring is continued for 18 hours. Potassium carbonate is then added until phase separation occurs and the triacetoneamine-N-oxide extracted four times with a total of 371.5g (4.52 mol) cyclohexene.
**b)** After addition of 1.01 g (4.5 mmol) cupric bromide the combined organic phases are brought to 60°C and 49g (0.38 mol) t-butylhydroperoxide slowly dosed in. The mixture is held at 60°C for a total of 2.3 hours. The greenish suspension is then cooled to 25°C followed by the addition of 280g aqueous 20% sodium sulfite solution. After stirring 1 hour the aqueous phase is split off, the organic phase washed with water and brine and then concentrated on a rotary evaporator.
**c)** The residue is dissolved in 1200ml methanol followed by the addition of 35.8g (0.49 mol) butylamine and 16.3g Pd on charcoal (10%). The mixture is stirred at 25°C during 1.5 hours and then hydrogenated at 50°C / 10 bar hydrogen during 1.5 hours. The mixture is filtered through Hyflo and the filtrate concentrated on a rotary evaporator. Distillation of the residue yields 112g (73%) of a yellow to slightly orange oil (bp 120°C / 0.8 mbar).

Analysis required for C₁₉H₃₈N₂O (310.53): C 73.49%, H 12.33%, N 9.02%; found: C 73.09%, H 12.04%, N 8.93%.
¹H-NMR (CDCl₃), δ (ppm): 0.9 (t, 3H), 1.1-1.6 (m, 24H), 1.7 (m, 4H), 2.0 (m, 2H), 2.6 (t, 2H), 2.7 (m, 1 H), 3.6 (m, 1 H).
¹³C-NMR (CDCl₃), δ (ppm): 14.04, 20.58, 21.17, 25.05, 25.97, 32.81, 32.84, 34.60, 46.74, 47.22, 48.20, 59.76, 81.69.

### Example 4: Synthesis of compound B' by reaction of NOR building block B with cyanuric chloride and ethanolamine:

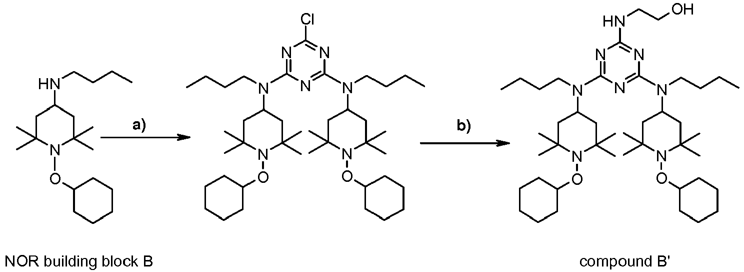

**a)** A solution of 6.2g (20mmol) NOR building block B in 10g cyclohexane is slowly added at 25°C to a stirred suspension of 1.9g (10mmol) cyanuric chloride in 8.4g cyclohexane. Stirring is continued for 30 minutes followed by the addition of 2.7g (20.4mmol) aqueous 30% NaOH solution. The mixture is heated to 70°C and stirred until the reaction is complete. The mixture is cooled down to 25°C, filtered, the aqueous phase split off and the organic phase washed with brine and concentrated on a rotary evaporator.
**b)** Excess ethanolamine (4g, 65mmol) is added and the solution heated to 110°C. Stirring is continued until the reaction is complete. The mixture is cooled down to 25°C, ethanolamine split off after addition of cyclohexane and the cyclohexane phase washed and evaporated to give a white powder.

Analysis required for C₄₃H₈₀N₈O₃ (757.17): C 68.21%, H 10.65%, N 14.80%; found: C 68.37%, H 10.60%, N 14.05%.

The as-prepared product exhibits higher quality compared to state-of-the-art material in terms of monomer content and transmission:

| compound B' | HPLC [Area%]^{a )} | Transmission [%]^{b)} |
|---|---|---|
| State of the art (Tinuvin® 152; CAS-no. 150686-79-6)) | 53 | 85.5 |
| prepared according to Example 4 (reactant NOR building block B not distilled) | 42 | 76.4 |
| prepared according to Example 4 (reactant NOR building block B distilled) | 80 | 88.6 |

| | | |
|---|---|---|
| ^{a)} Area of product (monomer) peak (retention time 28min) relative to sum of peaks; conditions: AAD-0004/2 with modified column (ZORBAX Extend C-18 column, 4.6mm x 250mm / 5µm, AGILENT No. 770450-902; column exhibiting enhanced stability at high pH); ^{b)} 425nm, 10% w/v solutions in m-xylene. | | |

### Example 5: Synthesis of compound C' by reaction of NOR building block B with cyanuric chloride and N,N'-bis(3-aminopropyl)ethylenediamine:

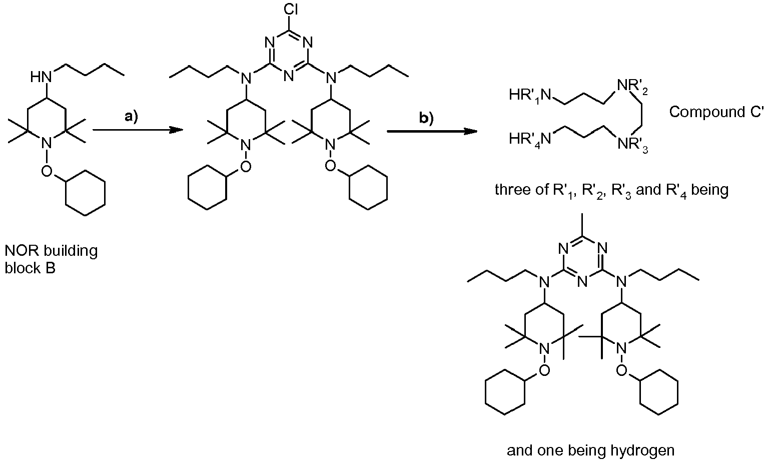

**a)** A solution of 6.5g NOR building block B in 10g cyclohexane is slowly added at 25°C to a stirred suspension of 1.9g (10mmol) cyanuric chloride in 10g cyclohexane. Stirring is continued for 30 minutes followed by the addition of 2.7g (20.4mmol) aqueous 30% NaOH solution. The mixture is heated to 70°C and stirred until the reaction is complete. The mixture is cooled down to 25°C, filtered, the aqueous phase split off and the organic phase washed with brine and concentrated on a rotary evaporator.
**b)** A mixture of 6g (8.2mmol) of the above crude product, 0.47g (2.7mmol) N,N'-bis(3-aminopropyl)ethylenediamine and 1.7g (8.5mmol) aqueous 20% NaOH solution is heated in a glass pressure bottle to 125°C during 17.5 hrs. The mixture is cooled down to 25°C, diluted with cyclohexane and the aqueous phase split off. The organic phase is brine washed and concentrated on a rotary evaporator. The crude oil is slowly added to boiling methanol, yielding a white precipitate. The suspension is treated with an Ultraturrax, filtered and the filtercake dried to yield the product as a white powder.

Analysis required for C₁₃₁H₂₄₁N₂₅O₆ (2262.51): C 69.54%, H 10.74%, N 15.48%; found: C 69.56%, H 10.60%, N 15.25%.

The as-prepared product exhibits higher quality compared to state-of-the-art material in terms of transmission:

| Compound C' | Transmission [%]^{b} | | |
|---|---|---|---|
| | 425nm | 450nm | 500nm |
| State of the art (Flamestab® NOR 116; CAS-no. 191680-81-6) | 68 | 75 | 84 |
| prepared according to Example 5 | 81 | 88 | 94 |

### Example 6: Preparation of NOR building block C in two steps from triacetonamine N-oxide:

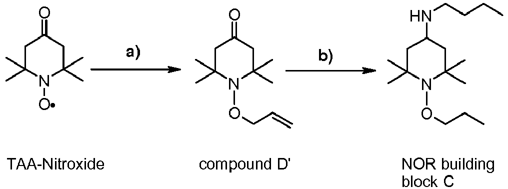

**a)** In a 300ml stainless steel autoclave are added 5.01g (29.45mmol) triacetone amine N-oxide, 198mg (0.9mmol) CuBr₂ and 286mg (0.9mmol) Bu₄NBr. The autoclave is sealed and 38.6g (920mmol) of propylene are added. The reaction is heated to 70°C (pressure ca 28 bar). When the temperature is reached, 7.6g (58.8mmol) of t-BuOOH (aqueous 70%) are added during 2.5 hours. The reaction is stirred for an additional 2 hours. The measured oxygen concentration of the gas phase is uncritical (<5%) throughout the reaction. Then the pressure in the autoclave is released. The autoclave is unloaded and rinsed with 50ml of dichloromethane. GLC analysis of the reaction mixture reveals about 90% conversion. The solvents are removed from the reaction mixture and the crude product (6.9g) purified by flash chromatography (silica gel, hexane / ethylacetate 3/1). Yield 4.1g (66%) of a white solid (mp 50 - 51°C; bp ca 80°C / 1mbar).
   Analysis required for C₁₂H₂₁NO₂ (211.31): C 68.21%, H 10.02%, N 6.63%; found: C 68.76%, H 10.15%, N 6.55%.
   ¹H-NMR (400MHz, CDCl₃), δ (ppm): 1.18 (s, 6H), 1.31 (s, 6H), 2.22 (d, J = 12.8Hz, 2H), 2.57 (d, J = 12.8Hz, 2H), 4.38 (d x t, J = 5.6Hz / 1.2Hz, 2H), 5.17 (d x q, J = 10.6Hz / 1.6Hz, 1H), 5.30 (d x q, J = 17.4Hz / 1.6Hz, 1 H), 5.88 - 5.95 (m, 1 H).
   ¹³C-NMR (100MHz, CDCl₃), δ (ppm): 22.4 (2 *C*H₃), 32.4 (2 *C*H₃), 53.2 (2 *C*H₂), 62.9 (2 CN), 78.4 (O*C*H₂), 116.6 (*C*H₂), 133.3 (CH), 207.8 (CO).
   LC/MS (m/z): 212 (MH⁺)
**b)** A mixture of 86.7g (0.41 mol) compound D', 35.2g (0.476mol) butylamine and 0.8g 10% Pt on charcoal is hydrogenated over night at 80°C and 50 bar. Filtration and evaporation of volatiles yields 104.2g (93.9%) of a slightly yellow oil.

Analysis required for C₁₆H₃₄N₂O (270.46): C 71.06%, H 12.67%, N 10.36%; found: C 70.86%, H 12.54%, N 10.49%.
¹H-NMR (400MHz, CDCl₃), δ (ppm): 0.93 (q, 6H), 1.17 (s, 6H), 1.19 (s, 6H), 1.2-1.31 (m, 2H), 1.32-1.37 (m, 2H), 1.41-1.47 (m, 2H), 1.51-1.56 (m, 2H), 1.71-1.74 (m, 2H), 2.59 (t, 2H), 2.73-2.78 (m, 1 H), 3.69 (t, 2H).
¹³C(DEPT)-NMR (100MHz, CDCl₃), δ (ppm): 10.95 (*C*H₃), 14.03 (*C*H₃), 20.6 (*C*H₂), 21.0 (*C*H₃), 21.8 (*C*H₂), 32.8 (*C*H₂), 33.3 (*C*H₃), 46.8 (*C*H₂), 48.2 (CH), 59.8 (C), 78.4 (*C*H₂).

### Example 7: Preparation of NOR building block D in one step

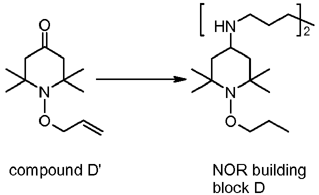

A mixture of 279g (1.32mol) compound D', 71.8g (0.6mol) 1,6-diaminohexane, 420ml ethanol and 1.2g 10% Pt on charcoal is hydrogenated over night at 100°C and 50 bar. The reaction mixture is filtered and volatiles evaporated to yield 315.5g (100%) of a slightly orange, viscous oil.

Analysis required for C₃₀H₆₂N₄O₂ (510.85): C 70.54%, H 12.23%, N 10.97%; found: C 70.47%, H 12.39%, N 10.94%.
¹H-NMR (400MHz, CDCl₃), δ (ppm): 0.95 (t, 6H), 1.15 (s, 12H), 1.18 (s, 12H), 1.20-1.26 (m, 4H), 1.34-1.36 (br m, 4H), 1.46-1.49 (m, 4H), 1.51-1.58 (m, 4H), 1.72-1.75 (m, 4H), 2.60 (t, 4H), 2.75-2.80 (m, 2H), 3.71 (t, 4H).
¹³C(DEPT)-NMR (100MHz, CDCl₃), δ (ppm): 10.95 (*C*H₃), 20.95 (*C*H₃), 21.96 (*C*H₂), 27.38 (*C*H₂), 30.57 (*C*H₂), 33.24 (*C*H₃), 46.63 (*C*H₂), 46.98 (*C*H₂), 48.14 (CH), 59.73 (C), 78.45 (*C*H₂).

### Example 8: Reaction of cyanuric chloride with NOR building blocks C and D

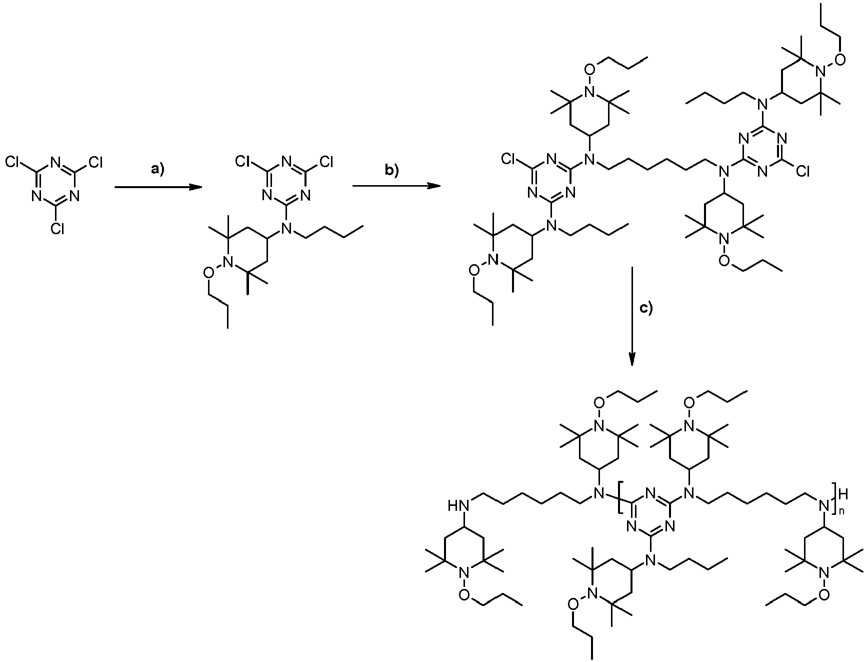

**a)** To a suspension of 24g (0.13mol) cyanuric chloride in 125ml xylene are slowly added at 5-10°C 35.2g (0.13mol) NOR building block C. The mixture is allowed to warm up to 40°C followed by the addition of 29g (0.145mol) NaOH (aqueous 20%). After stirring for one hour at 40°C, a sample is withdrawn and analyzed. GLC indicates >90% conversion. The structure is confirmed by NMR.
**b)** The aqueous phase is split off and the organic phase heated to 70°C followed by the slow addition of 33.2g (0.065mol) melted NOR building block D and 33g water. After addition of NaOH (aqueous 30%, 20g, 0.15mol) the mixture is brought to 80°C where it is left for one hour. The structure is confirmed by NMR.
**c)** The hot aqueous phase is split off. The organic phase is cooled down to 25°C and transferred into an autoclave. After addition of 66.4g (0.13mol) NOR building block D and 28.6g (0.143mol) NaOH (aqueous 20%) the autoclave is sealed and heated to 175°C where it is left for 4 hours. After cooling down to 25°C the autoclave is unloaded and the aqueous phase split off (at 80°C). The structure is confirmed by NMR. Mn / Mw (GPC) 1700 / 3300 - 1900 / 3800. Amount of residual NOR building block D ca 10% (area%).
**d)** Further reaction with 2-chloro-4,6-bis(dibutylamino)-s-triazine yields:

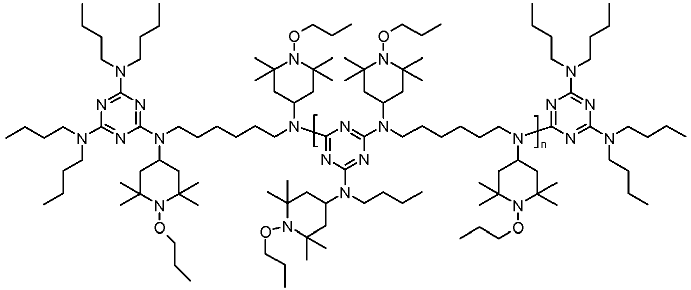

### Example 9: Preparation of compound D'

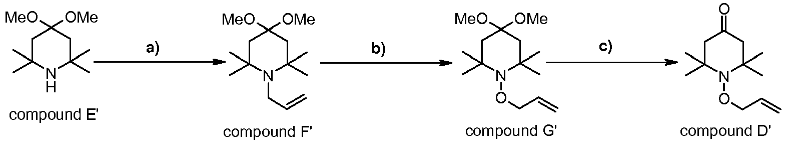

**a)** A mixture of 15.1g (75mmol) compound E' (synthesized according to EP748849, Rhone-Poulenc), 20g (150mmol) NaOH (aqueous 30%), 1.27g (3.7mmol) Bu₄NHSO₄ and 18.3g (151.3mmol) allylbromide is stirred at 90°C during 24 hours (95% conversion by GLC). The mixture is cooled down to 25°C followed by the addition of toluene (20ml). The aqueous phase is split off and the organic phase concentrated on a rotary evaporator. Distillation of the residue affords 8.1 g (45%) of a slightly yellow oil.
   Analysis required for C₁₄H₂₇NO₂ (241.38): C 69.67%, H 11.27%, N 5.80%; found: C 69.62%, H 10.95%, N 5.77%.
   ¹H-NMR (300MHz, CDCl₃), δ (ppm): 1.10 (s, 12H), 1.72 (s, 4H), 3.18-3.21 (m, 2H), 3.19 (s, 6H), 4.94 (d x q, J = 10.2 Hz / 2Hz, 1H), 5.16 (d x q, J = 17.1 Hz / 2Hz, 1H), 5.80-5.92 (m, 1H).
**b)** To a mixture of 23g (95mmol) compound F' and 19.28g (182mmol) Na₂CO₃ in 200ml toluene is added at -5°C 20.48g (105mmol) AcOOH (39% in AcOH) during 40 minutes. The mixture is stirred at 0°C (6 hours; 83% conversion by GLC) and then filtered. The filtrate is washed with NaOH 1M (3x20ml) and brine (3x20ml). The organic phase is dried (Na₂SO₄), filtered and the solvent evaporated. The residue is flash-filtrated over silicagel (hexane) to afford, after evaporation of the solvent, 15g (61 %) of a yellow liquid.
   Analysis required for C₁₄H₂₇NO₃ (257.38): C 65.33%, H 10.57%, N 5.44%; found: C 65.48%, H 10.80%, N 5.33%.
   ¹H-NMR (400MHz, CDCl₃), δ (ppm): 1.10 (s, 6H), 1.27 (s, 6H), 1.59 (d, J = 13Hz, 2H), 1.94 (d, J = 13Hz, 2H), 3.17 (s, 6H), 4.30 (d x t, J = 5.2Hz / 1.6Hz, 2H), 5.14 (d x q, J = 10.4Hz / 1.6Hz, 1 H), 5.29 (d x q, J = 17.4Hz / 1.6Hz, 1 H), 5.86-5.96 (m, 1 H).
**c)** A solution of 1g (3.9mmol) compound G', 1g water and one drop (pasteur pipette) HCl (aqueous 32%) in 6ml THF is stirred at 25°C. After 4 hours (97% conversion by GLC) NaHCO₃ is added, the mixture filtrated and the filtrate concentrated on a rotary evaporator. The residue is extracted with hexane to afford, after evaporation of the solvent, 0.62g (75%) of a white solid.

¹H-NMR: same as in Example 6 a.

### Example 10: Preparation of compound D' using an oxidation methodology published by H. Adam et al., J. Org. Chem. 61, 1467-1472 (1996)

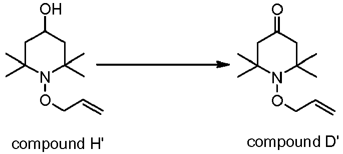

To a mixture of 5.05g (23.7mmol) compound H' (synthesized according to Ciba patent DE19907945), 1.03g (2.7mmol) Zr(OtBu)₄ and 9.5g activated molecular sieve (4A) in 45ml toluene are slowly added at 25°C 10.66g (47.3mmol) t-BuOOH (40% in cyclohexane). The mixture is stirred at 25°C for 24 hours (86% conversion by GLC) and then washed with saturated aqueous sodium potassium tartrate and brine. The aqueous phase is split off and the organic phase dried (Na₂SO₄). Evaporation of the solvent yields 3g of a slightly orange solid, which is analyzed by 400MHz ¹H-NMR adding 4,4'-di-tert-butylbiphenyl as internal standard. Yield calculated based on NO-C*H₂*CH=CH₂ (δ = 4.38ppm) 2.1g (42%).

¹H-NMR: same as in Example 6 a.

### Example 11: Preparation of compound G'

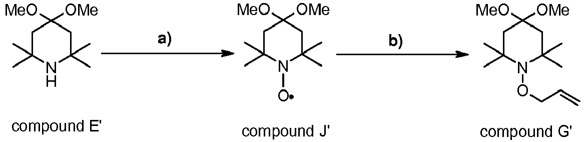

**a)** To a mixture of 2.1g (10mmol) compound E' and 0.16g (0.48mmol) Na₂WO₄x2H₂O in 10ml water are slowly added at 5°C 2.7g (24mmol) H₂O₂ (aqueous 30%). The mixture is stirred at 25°C until the starting material had disappeared (6 hours). Diethylether (20ml) is added and the aqueous phase saturated with K₂CO₃. The aqueous phase is split off and washed with diethylether. The organic phases are combined, the solvent evaporated and the residue dried on an oil pump to afford 2.15g (99%) of a red liquid.
   Analysis required for C₁₁H₂₂NO₃ (216.30): C 61.08%, H 10.25%, N 6.48%; found: C 61.03%, H 10.08%, N 6.39%.
**b)** Compound G' is synthesized in analogy to example 6a from 6.35g (29.4mmol) compound J', 38.6g (920 mmol) propylene, 0.328g (0.9 mmol) Bu₄NI and 7.6g (58.8mmol) t-BuOOH (aqueous 70%). GLC analysis of the reaction mixture reveals about 50% conversion. Non-reacted CG43-0819 is separated off by flash-chromatography (silica gel, hexane / ethylacetate 8 / 2) and the dried residue analyzed by 400MHz ¹H-NMR adding 4,4'-di-tert-butylbiphenyl as internal standard. Yield calculated based on NO-C*H₂*CH=CH₂ (δ = 4.30ppm) 1.5g (20%).

¹H-NMR: same as in Example 9 b. LC/MS (m/z): 258 (MH⁺)

### Example 12: Preparation of compound K' by oxidation of Chimassorb® 2020:

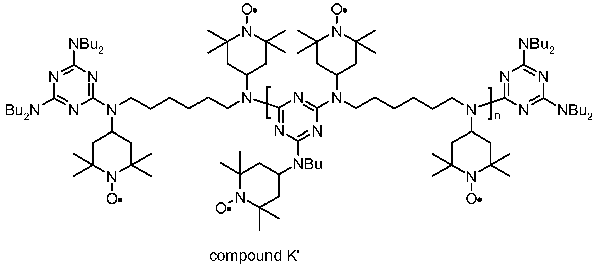

To a mixture of 20g Chimassorb® 2020 (commercially available from Ciba Specialty Chemicals; Mₙ by GPC: 2819 g/mol, ca. 3.5meq NH/g; CAS-no. 192268-64-7) and 35.5g (336.5mmol) Na₂CO₃ in 40ml of CH₂Cl₂ are slowly added at -5°C 26.6g (136.5mmol) of AcOOH (39% in AcOH). The mixture is kept stirable by concomitant, slow addition of a total of 90ml of water. The mixture is then stirred overnight at 20°C and the organic phase split off. The aqueous phase is extracted with CH₂Cl₂ and the combined organic phases washed with NaOH and brine, dried over MgSO₄ and the solvent evaporated to afford 18g of a red powder.

Analysis: found C 65.17%, H 10.00%, N 16.84%, O 6.64%.

### Example 13: Preparation of compound L' from compound K' via t-BuOOH hydrogen abstraction from propylene:

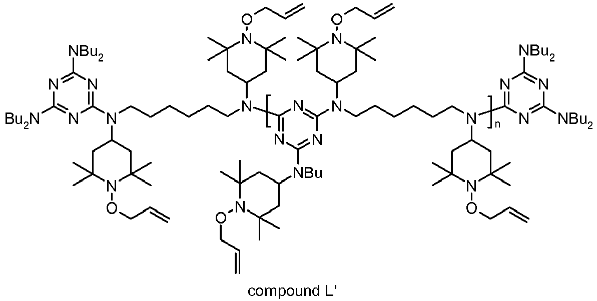

An autoclave is charged with 2.23g compound K' (ca 3.3meq NO·/g), 0.081g (0.22mmol) Bu₄Nl and 10ml chlororbenzene. The autoclave is sealed followed by the addition of 19.3g (458.6mmol) propylene. The system is then brought to 70°C (ca 22bar), whereafter 2.85g (22.1mmol) t-BuOOH (aqueous 70%) are slowly pumped in during 2 hours. The reaction mixture is held for another 30 minutes at 70°C and then cooled to 25°C (ca 10bar). Pressure is released and the autoclave uncharged. Volatiles are removed on a rotary evaporator and the residue dried, affording compound L' as yellowish powder.

¹H-NMR (300MHz, CDCl₃), δ (ppm, NO-C*H₂*CHCH₂ only): 4.3 (br s)

## Claims

1. A process for the preparation of a compound of formula (300) 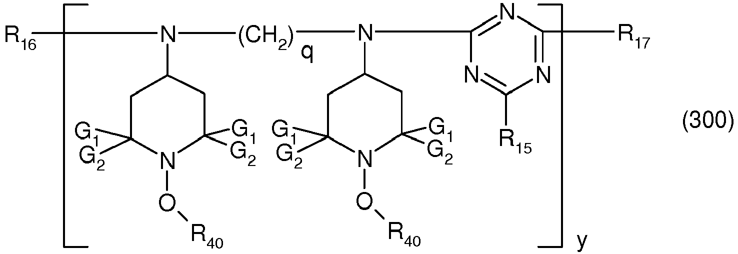 wherein
G₁ and G₂ are independently C₁-C₄alkyl;
R₄₀ is propyl or 2-propenyl;
y is 2 to 20;
q is 2 to 8;
R₁₅ is morpholino, piperidino, 1-piperizinyl, alkylamino of 1 to 8 carbon atoms, -N(C₁-C₈alkyl)T₁₀, or -N(alkyl)₂ of 2 to 16 carbon atoms, T₁₀ is 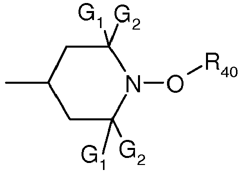
R₁₆ is hydrogen, C₂-C₄acyl, carbamoyl substituted by C₁-C₄alkyl, s-triazinyl substituted once by chlorine and once by R₁₅, or s-triazinyl substituted twice by R₁₅ with the condition that the two R₁₅ substituents may be different;
R₁₇ is chlorine, amino substituted by C₁-C₈alkyl or by T₁₀, -N(C₁-C₈alkyl)T₁₀, -N(alkyl)₂ of 2 to 16 carbon atoms, or the group T₁₃ 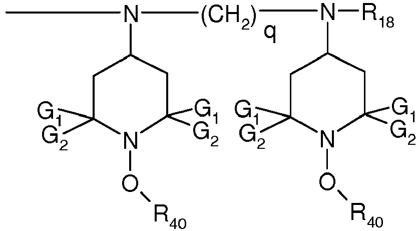 R₁₈ is hydrogen, C₂-C₄acyl, carbamoyl substituted by C₁-C₄alkyl, s-triazinyl substituted twice by -N(alkyl)₂ of 2 to 16 carbon atoms or s-triazinyl substituted twice by -N(C₁-C₈alkyl)T₁₀;
which comprises oxidizing a compound of formula (300) wherein >N-O-R₄₀ is >N-H to a compound of formula (300) wherein -O-R₄₀ is -O•, which is subsequently reacted with propene;
and hydrogenating this compound for obtaining a compound of formula (300) with R₄₀ = propyl.

2. A process according to claim 1, wherein G₁ and G₂ are methyl.

## Patentansprüche

1. Verfahren zur Herstellung einer Verbindung der Formel (300) 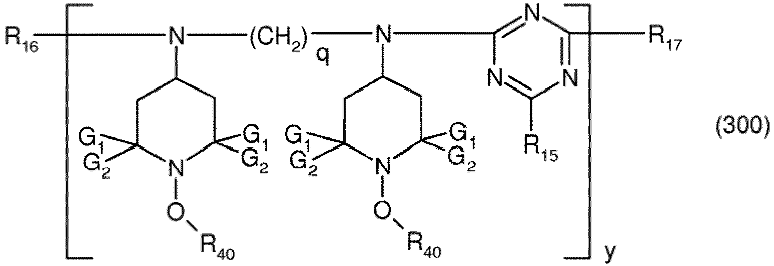 in der
G₁ und G₂ unabhängig für C₁-C₄-Alkyl stehen;
R₄₀ für Propyl oder 2-Propenyl steht;
y für 2 bis 20 steht;
q für 2 bis 8 steht;
R₁₅ für Morpholino, Piperidino, 1-Piperizinyl, Alkylamino mit 1 bis 8 Kohlenstoffatomen, -N(C₁-C₈-Alkyl)T₁₀ oder -N(Alkyl)₂ mit 2 bis 16 Kohlenstoffatomen steht, T₁₀ für 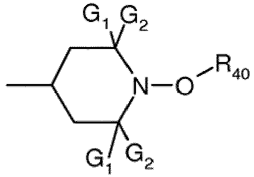 steht;
R₁₆ für Wasserstoff, C₂-C₄-Acyl, Carbamoyl, das durch C₁-C₄-Alkyl substituiert ist, s-Triazinyl, das einfach durch Chlor und einfach durch R₁₅ substituiert ist, oder s-Triazinyl, das zweifach durch R₁₅ substituiert ist, mit der Maßgabe, dass die zwei R₁₅-Substituenten verschieden sein können;
R₁₇ für Chlor, Amino, das durch C₁-C₈-Alkyl oder durch T₁₀ substituiert ist, -N(C₁-C₈-Alkyl)T₁₀, -N(Alkyl)₂ mit 2 bis 16 Kohlenstoffatomen oder die Gruppe T₁₃ 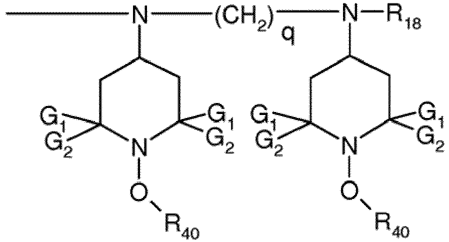 steht;
R₁₈ für Wasserstoff, C₂-C₄-Acyl, Carbamoyl, das durch C₁-C₄-Alkyl substituiert ist, s-Triazinyl, das zweifach durch -N(Alkyl)₂ mit 2 bis 16 Kohlenstoffatomen substituiert ist, oder s-Triazinyl, das zweifach durch -N(C₁-C₈-Alkyl)T₁₀ substituiert ist, steht;
bei dem man eine Verbindung der Formel (300), in der >N-O-R₄₀ für >N-H steht, zu einer Verbindung der Formel (300), in der -O-R₄₀ für -O• steht, oxidiert, die anschließend mit Propen umgesetzt wird,
und diese Verbindung zum Erhalt einer Verbindung der Formel (300) mit R₄₀ = Propyl hydriert.

2. Verfahren nach Anspruch 1, bei dem G₁ und G₂ für Methyl stehen.

## Revendications

1. Procédé de préparation d'un composé de formule (300) 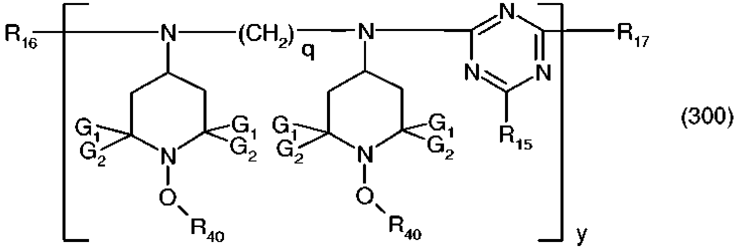 dans lequel
G₁ et G₂ sont indépendamment alkyle en C₁-C₄ ;
R₄₀ est propyle ou 2-propényle ;
y est 2 à 20 ;
q est 2 à 8 ;
R₁₅ est morpholino, pipéridino, 1-pipérizinyle, alkylamino de 1 à 8 atomes de carbone, -N(alkyle en C₁-C₈)T₁₀, ou -N(alkyle)₂ de 2 à 16 atomes de carbone, T₁₀ est 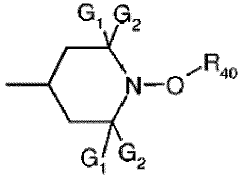
R₁₆ est hydrogène, acyle en C₂-C₄, carbamoyle substitué par alkyle en C₁-C₄, s-triazinyle substitué une fois par un chlore et une fois par R₁₅, ou s-triazinyle substitué deux fois par R₁₅ à condition que les deux substituants R₁₅ puissent être différents ;
R₁₇ est chlore, amino substitué par alkyl en C₁-C₈ ou par T₁₀, -N (alkyle en C₁-C₈)T₁₀, -N(alkyle)₂ de 2 à 16 atomes de carbone, ou le groupe T₁₃ 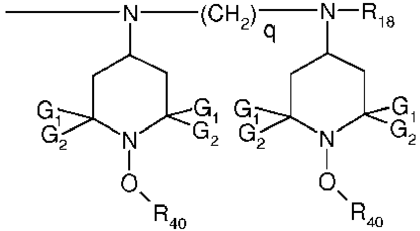 R₁₈ est hydrogène, acyle en C₂-C₄, carbamoyle substitué par alkyle en C₁-C₄, s-triazinyle substitué deux fois par -N(alkyle)₂ de 2 à 16 atomes de carbone ou s-triazinyle substitué deux fois par -N(alkyle en C₁-C₈)T₁₀ ;
qui comprend l'oxydation d'un composé de formule (300) dans lequel >N-O-R₄₀ est >N-H en composé de formule (300) dans lequel -O-R₄₀ est -O•, qui réagit ensuite avec le propène ;
et l'hydrogénation de ce composé pour obtenir un composé de formule (300) avec R₄₀ = propyle.

2. Procédé selon la revendication 1, dans lequel G₁ et G₂ sont méthyle.
